# EUROPEAN PATENT SPECIFICATION

(11) **EP 3 569 275 B1**
(45) Date of publication and mention of the grant of the patent: **10.11.2021**
(21) Application number: 19174758.3
(22) Date of filing: 15.05.2019
(51) Int. Cl.: A61M 15/06, A24F 47/00, A61M 11/04, A61M 15/02

(54) **MICROFLUIDIC DISPENSER DEVICE FOR DELIVERING INHALABLE SUBSTANCES**
MIKROFLUIDISCHE SPENDERVORRICHTUNG ZUR ABGABE VON INHALIERBAREN SUBSTANZEN
DISPOSITIF DE DISTRIBUTION MICROFLUIDIQUE POUR L'ADMINISTRATION DE SUBSTANCES INHALABLES

(30) Priority: 15.05.2018 IT 201800005372
(43) Date of publication of application: 20.11.2019
(73) Proprietor: STMicroelectronics S.r.l., 20864 Agrate Brianza (MB) (IT)
(72) Inventor: DI MARCO, Oriana Rita Antonia, 20147 MILANO (IT); GIUSTI, Domenico, 20867 CAPONAGO (MB) (IT)
(74) Representative: Bernotti, Andrea

(56) References cited:
- EP-A1- 1 452 199
- EP-A1- 1 552 857
- EP-A2- 1 306 219
- EP-A2- 1 317 939
- WO-A1-2009/072669
- WO-A1-2015/066127
- WO-A1-2016/145072
- US-A1- 2003 186 474
- US-A1- 2006 060 191
- US-A1- 2009 260 624
- US-A1- 2018 036 763
- US-B1- 9 174 445

## Description

The present invention relates to a microfluidic dispenser device for delivering inhalable substances.

As is known, the need to control precisely delivery of inhalable substances, both for therapeutic purposes and for the production of non-medical devices, such as the so-called electronic cigarettes, has led to the development of miniaturised delivering devices that are easy to use.

A dispenser device of inhalable substances of a known type normally comprises a tank, which contains a fluid with the substances to be delivered in solution, and at least one delivery chamber, provided with ejector nozzles and supplied by the tank. An actuator housed in the chamber driven by a control device causes expulsion of a controlled amount of fluid.

Currently, in particular in electronic cigarettes, actuators of a resistive or inductive type are mainly used.

In resistive actuators, a resistive electrode is placed within the chamber and is wound, in contact, around a spongy cylindrical body, also referred to as "wick", which is generally made of glass fibre. The electrode traversed by current heats the fluid to be delivered up to boiling point, and the formation of bubbles causes expulsion of corresponding volumes of fluid to be delivered. Control of delivery is generally based upon flowmeters that detect the flow rate of fluid coming out of the chamber. Delivery devices of this sort suffer from certain limitations. In the first place, the electrode is directly in contact with the fluid to be delivered, and this may lead to release of undesirable and potentially harmful substances. Moreover, the entire volume of fluid present in the chamber is brought to boiling point and hence reaches rather high temperatures. Such a condition may trigger reactions in the fluid that alter the substances to be delivered. If the substances to be delivered are drugs, the reactions may render the active principles inefficacious. If, instead, the device delivers surrogates of smoke, the organoleptic characteristics may be degraded. In the worst scenarios, the reactions due to the high temperature may produce harmful substances that are inhaled to the detriment of the user. A further limit of known devices is the low precision in controlling the doses of inhalable substances delivered.

In actuators of an inductive type, a coil is wound around the wick, at a distance. The coil traversed by current remains relatively cold, but generates a magnetic field that heats the spongy body and the liquid until it causes expulsion of the latter. Actuators of this type do not present problems linked to the release of substances from the coil (which is not in contact with the liquid) and to the excessive heating, but are costly and not suited to being integrated in disposable cartridges, as would be preferable.

US 9 174 445_B1 discloses a microfluidic dispenser device for delivering inhalable substances comprising a casing, a driving circuit, housed in the casing, and a microfluidic cartridge, housed in the casing and having a tank containing a liquid to be delivered. The microfluidic cartridge has a nebuliser controlled by the driving device and in turn comprising a substrate and a chamber layer on the substrate. Chambers are formed on the substrate in the chamber layer and are fluidically coupled to the tank for receiving the liquid to be delivered. Heaters controlled by the driving device are formed on the substrate in positions corresponding to respective chambers, are thermally coupled to the respective chambers, and are separated from the respective chambers by an insulating layer. Nozzles fluidically connect respective chambers to the outside of the nebuliser. The nebuliser has supply passages fluidically coupled to the tank and the chambers are fluidically coupled to respective supply passages through respective microfluidic channels. Moreover, the chambers are aligned along edges of the supply passages and are evenly distributed.

Other examples of known microfluidic dispenser devices are disclosed in WO 2016/145072 A1, WO 2015/066127 A1, US 2009/260624 A1, EP 1 317 939 A2, EP 1 306 219 A2 and EP 1 552 857 A1.

The aim of the present invention is to provide a microfluidic dispenser device for delivering inhalable substances that will enable the limitations described above to be overcome or at least mitigated.

According to the present invention, a microfluidic dispenser device for delivering inhalable substances is provided, as defined in Claim 1.

For a better understanding of the invention, some embodiments thereof will now be described, purely by way of non-limiting example and with reference to the attached drawings, wherein:
- Figure 1 is a perspective view of a microfluidic dispenser device of inhalable substances according to an embodiment of the present invention;
- Figure 2 is a perspective view of the microfluidic delivery device of Figure 1, sectioned along the plane II-II of Figure 1;
- Figure 3 is a perspective view of a microfluidic cartridge used in the microfluidic delivery device of Figure 1;
- Figure 4A is a side view of the microfluidic cartridge of Figure 3, sectioned along the plane IV-IV of Figure 3;
- Figure 4B is a side view of a microfluidic cartridge in accordance with a different embodiment of the present invention;
- Figure 5 is a perspective view of a component of the microfluidic cartridge of Figure 3;
- Figure 6 is an exploded perspective view of the component of Figure 5;
- Figure 7 is an enlarged perspective view of a portion of the component of Figure 5, with parts removed for clarity;
- Figure 8 is a top plan view of an enlarged detail of the component of Figure 5;
- Figure 9 is a perspective view of the detail of Figure 8, sectioned along the plane IX-IX of Figure 8;
- Figure 10 is a perspective view of a detail of a component of a microfluidic cartridge that can be used in a microfluidic delivery device according to a different embodiment of the invention;
- Figure 11 is a perspective view of a detail of a component of a microfluidic cartridge that can be used in a microfluidic delivery device according to a further embodiment of the invention;
- Figure 12 is a perspective view of a detail of a component of a microfluidic cartridge that can be used in a microfluidic delivery device according to a further embodiment of the invention;
- Figures 13A-13E illustrate operation of the component of Figure 5;
- Figure 14 is a perspective cross-sectional view of a component of a microfluidic cartridge that can be used in a microfluidic delivery device according to a further embodiment of the invention;
- Figure 15 is a top plan view of an enlarged detail of the component of Figure 14; and
- Figure 16 is a schematic lateral sectional view of a microfluidic delivery device according to a further embodiment of the invention.

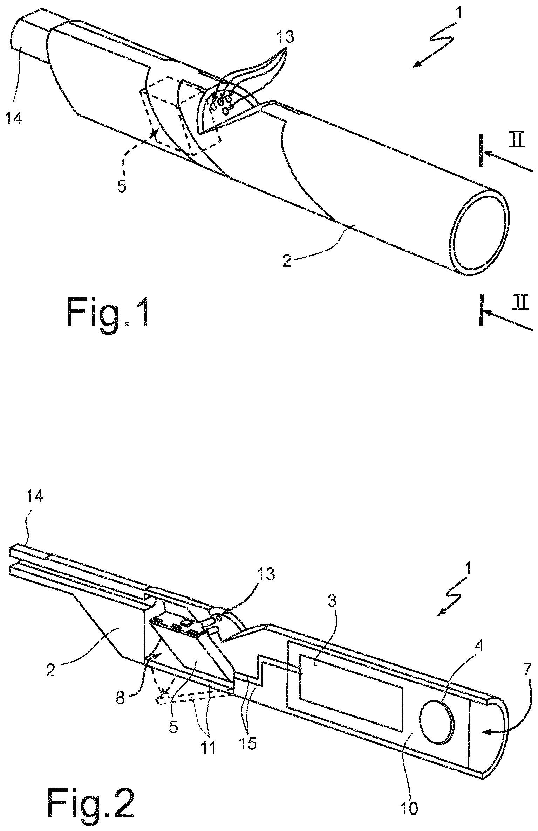

With reference to Figures 1 and 2, number 1 designates as a whole a microfluidic dispenser device for delivering inhalable substances, which in the embodiment illustrated is an electronic cigarette. The microfluidic delivery device 1 comprises a casing 2, housed within which are a driving device 3, a battery 4, and a disposable microfluidic cartridge 5.

In greater detail, the casing 2 comprises an elongated tubular body 6 made of polymeric and/or metal material, in which a control housing 7 and a cartridge housing 8 are obtained. In one embodiment, the control housing 7 is a substantially axial blind cavity, which is open at a first end 2a of the casing 2 and may be closed, for example, with an appropriately designed lid (not illustrated). The driving device 3 may be welded on a support 10, for example a PCB (Printed Circuit Board) that may be inserted in the control housing 7 together with the battery 4.

The cartridge housing 8 is defined by a chamber set between the control housing 7 and a second end 2b of the casing 2 and is accessible through a hatch 11 for insertion and removal of the cartridges 5. The cartridge housing 8 communicates with the outside through inlet holes 13 and a mouthpiece 14 for release of the inhalable substance. More precisely, the inlet holes 13 and the mouthpiece 14 are arranged so that suction through the mouthpiece 14 will cause recall of air into the cartridge housing 8 through the inlet holes 13, passage of the air drawn in through the cartridge housing 8, and subsequent release through the mouthpiece 14.

Electrical connection lines 15 are embedded in the casing 2 and extend between the control housing 7 and the cartridge housing 8 for electrically coupling the driving device 3 and the microfluidic cartridge 5 that is located in the cartridge housing 8.

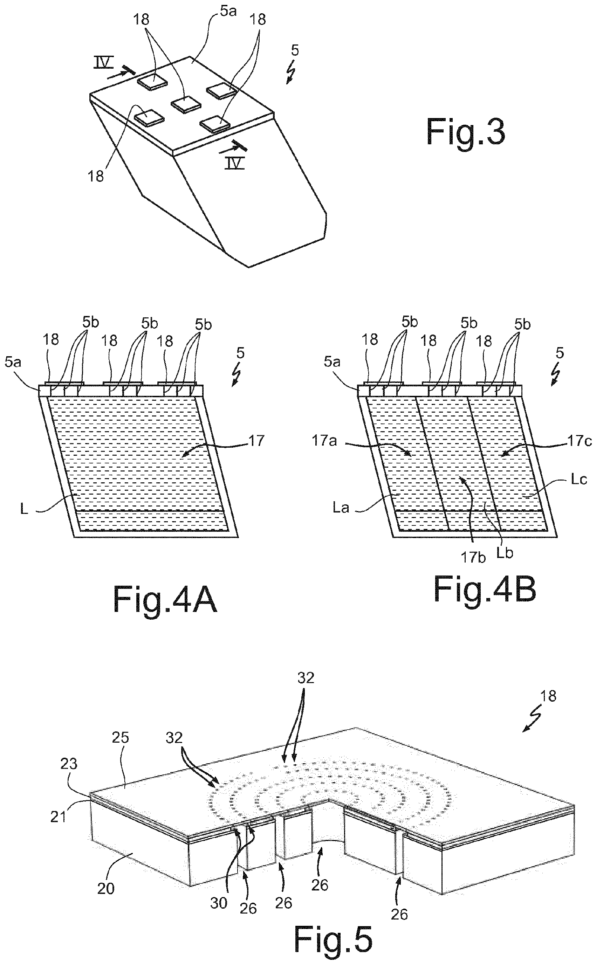

Figures 3 and 4A illustrate an example of microfluidic cartridge 5, which comprises a tank 17, containing a liquid L to be delivered, and a plurality of nebulisers 18 controlled by the driving device 3. The nebulisers 18 are bonded to an outer face of a lid 5a of the microfluidic cartridge 5, defined, for example, by a PCB and provided with through channels 5b for fluidic coupling of the nebulisers 18 to the tank 17. In the example of Figures 3 and 4A, in particular, the microfluidic cartridge 5 comprises five nebulisers 18 arranged in criss-cross fashion. The number and arrangement of the nebulisers 18 may, however, vary according to design preferences. In one embodiment, even just one nebuliser 18 may be present.

In an alternative embodiment, to which Figure 4B refers, the microfluidic cartridge 5 comprises a plurality of tanks, for example three tanks 17a-17c, separated from each other and containing respective liquids La-Lc with respective distinct substances to be delivered. Each tank 17a-17c is fluidly coupled with a respective nebulizer 18 or group of nebulizers 18, distinct from nebulizers 18 or groups of nebulizers 18 of the other tanks. In this way, it is possible to deliver several substances at the same time with controlled doses of each. The number and size of the tanks, as well as the type and number of substances to be delivered, may be selected according to project preferences.

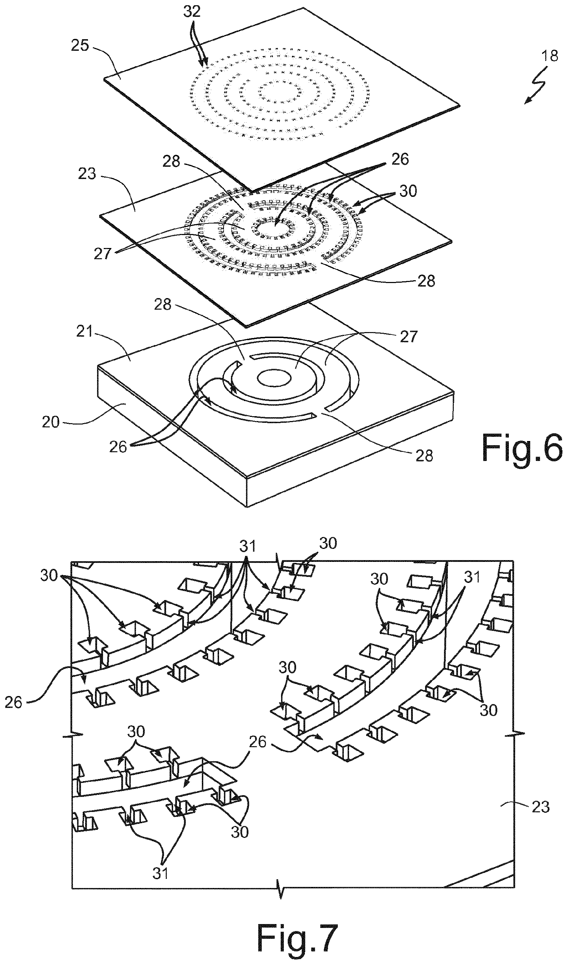

Figures 5 and 6 show in greater detail one of the nebulisers 18. It is understood that the other nebulisers 18 have the same structure as the one illustrated. In other embodiments, however, the nebulisers could differ as regards some details, such as the number and distribution of the ejection nozzles (described in greater depth hereinafter).

As may be appreciated more fully from the exploded view of Figure 6, the nebuliser 18 comprises a substrate 20, covered by an insulating layer 21, a chamber layer 23, which extends over the insulating layer 21, and a nozzle plate 25 bonded to the chamber layer 23. The substrate 20, the insulating layer 21, and the chamber layer 23 may, for example, be made, respectively, of semiconductor material, silicon oxide or silicon nitride, and a polymeric material, such as dry film. The nozzle plate 25 may be made of the same material that forms the chamber layer 23 or of semiconductor material.

Supply passages 26 fluidically coupled to the tank 17 are provided through the substrate 20, the insulating layer 21, and the chamber layer 23. In one embodiment, the supply passages 26 are circular and concentric and define annular frame regions 27 comprising respective portions of the substrate 20, of the insulating layer 21, and of the chamber layer 23, which are also concentric. In the embodiment illustrated in Figures 5 and 6, in particular, two outer supply passages 26 define two frame regions 27 and separate them from the rest of the substrate 20. The innermost supply passage 26 is arranged at the centre. Bridges 28 connect the frame regions 27 to one another and the outermost frame region 27 to the substrate 20.

It is, however, understood that the shape and number of the supply passages (and consequently of the substrate portions adjacent to the openings) may be freely defined according to the design preferences. By way of non-limiting example, the supply passages could have a generally polygonal or else rectilinear shape and be parallel to one another.

Chambers 30 are formed in the chamber layer 23 along the supply passages 26, as illustrated also in Figure 7. In the embodiment illustrated, the chambers 30 are aligned along the inner and outer edges of the supply passages 26 and are evenly distributed. Moreover, the chambers 30 are fluidically coupled to the supply passages 26 through respective microfluidic channels 31 and are delimited by the insulating layer 21 and, on the side opposite to the substrate 20, by the nozzle plate 25.

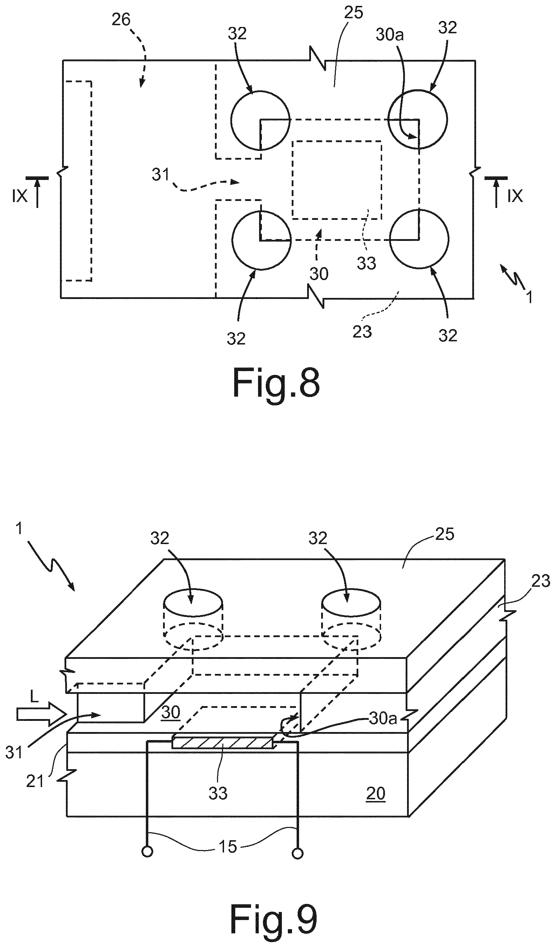

Figures 8 and 9 illustrate in greater detail one of the chambers 30. In the non-limiting example illustrated, all the chambers 30 have the same shape, structure, and size.

The chamber 30 has a parallelepipedal shape with an approximately rectangular base and is delimited laterally by walls 30a that define a lateral surface of the chamber 30 itself.

The chamber 30 is provided with nozzles 32 formed in the nozzle plate 25 in positions corresponding to respective corners of the chamber 30, so that portions of the surfaces of the walls 30a extend through the base area of the nozzles 32. The access to the nozzles 32 from the chamber 30 is hence partially obstructed, and the section of passage is a fraction of the base area of the nozzles 32. In the example illustrated, in particular, the area of the section of passage is approximately one quarter of the base area of the nozzles 32. In an alternative embodiment (not illustrated), both the chambers and the nozzles are provided in the chamber layer. More precisely, the chambers are formed on a first face of the chamber layer facing the substrate and occupy a portion of the thickness of the chamber layer itself. The nozzles extend for the remaining portion of the thickness of the chamber layer, between the respective chambers and a second face of the chamber layer opposite to the first face.

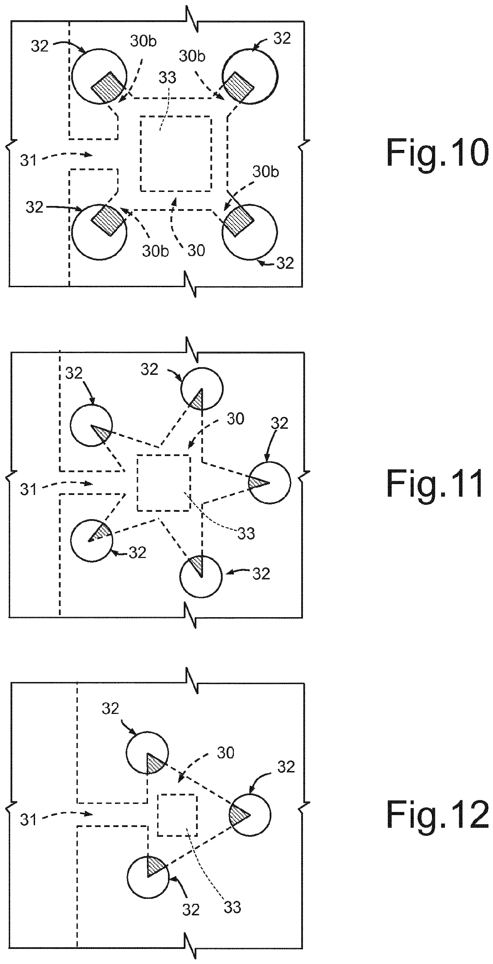

The shape of the chamber 30 and the arrangement of the nozzles 32 are not to be considered binding, but may be provided according to design preferences. Alternative examples, which are in any case non-limiting, of the chamber 30 and of the nozzles 32 are illustrated in Figures 10-12 (rectangular chamber 30 with niches 30b at the vertices and nozzles 32 partially overlapping the niches 30b, Figure 10; chamber 30 that is star-shaped, with nozzles 32 in positions corresponding to the points of the star shape, Figure 11; triangular chamber 30, with nozzles 32 at the vertices, Figure 12).

A heater 33 (Figure 8) is provided within the insulating layer 21 in a position corresponding to the chamber 30, and forms an actuator. The heater 33 may be made (by way of non-limiting example) of polycrystalline silicon, Al, Pt, TiN, TiAlN, TaSiN, TiW. A portion of the insulating layer 21, having a thickness such as to enable thermal coupling with the chamber 30, coats a face of the heater 33 that faces the chamber 30. Consequently, the heater 33 is separated from the chamber 30 and there is no direct contact between the heater 33 and the liquid present in the chamber 30. In one embodiment (not illustrated), the heater may be coated with a thin layer of an insulating and chemically inert material different from the material that forms the insulating layer 21 so as to obtain in any case thermal coupling with the chamber 30 and separation from the liquid L contained in the chamber 30. The heater 33 is controlled by the driving device 3, to which the heater 33 is connected through the electrical connection lines 15 (Figures 1 and 2), illustrated only schematically in Figure 8. The heater 33 may have an area of approximately 40 × 40 µm² and generate an energy of, for example, 3.5 µJ, and is able to reach a maximum temperature of 450°C in 2 µs.

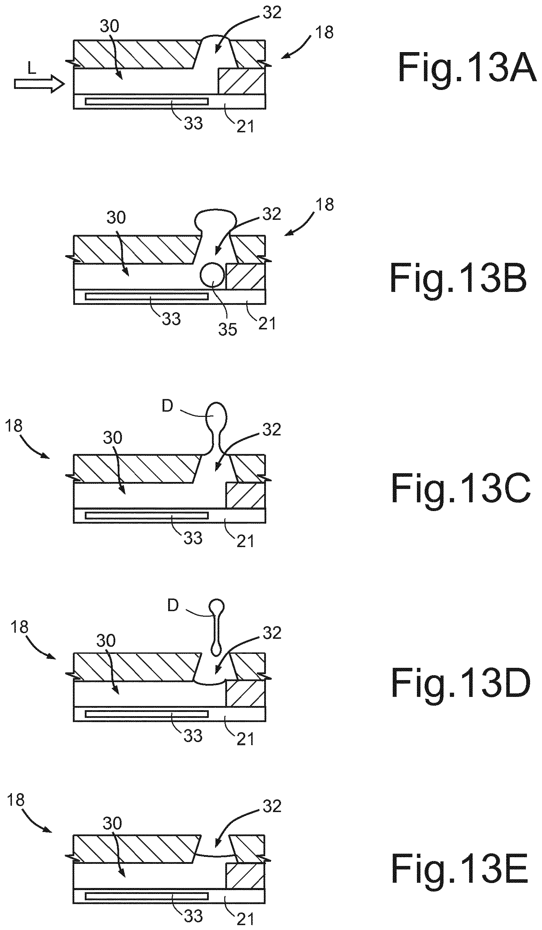

Operation of the nebuliser 18 is illustrated schematically in Figures 13A-13E. The liquid L reaches the chamber 30 from the tank 17, passing through the supply passages 26 and the microfluidic channels 31. The heater 33 is activated by the driving device 3 for some microseconds until it reaches a programmed temperature, for example 450°C. In this way, a layer of the liquid L of the thickness of some micrometres is rapidly heated, whereas the temperature of the rest of the liquid L present in the chamber 30 does not vary appreciably, owing to the delay in conduction of heat. The pressure in the layer of liquid L adjacent to the heater 33 increases to a high level, for example approximately 5 atm, to form a vapour bubble 35 (Figure 13B), which disappears after a few microseconds, for example 10-15 µs. The pressure thus generated pushes a drop D of liquid 18 through the nozzles 32, as illustrated in Figures 13C-13D, and then the liquid L present in the chamber 30 returns to the initial condition (Figure 13E).

The shape of the nozzles 32 and the area of the section of passage (which is determined by partial overlapping of the nozzles 32 and of the walls 30a of the chamber 30) are selected in such a way that the drops released have a desired diameter. Advantageously, the use of nozzles staggered with respect to the walls of the chambers enables reduction of the area of the sections of passage between the chambers and the nozzles and makes it possible to obtain drops having a very small diameter, as little as 1 µm, corresponding to a volume of approximately 0.0045 pl, without having to resort to sublithographic processing techniques.

The structure of the nebulisers 18, which can draw advantage from the precision of semiconductor manufacturing techniques, enables an extremely accurate control over the amount of nebulised liquid and, in other words, over the dosage of the substance to be inhaled that is released. Moreover, release is carried out without heating significantly the entire volume of liquid L present in a chamber 30. As has been discussed, in fact, it is sufficient to bring to a high temperature a rather thin layer of liquid L to create a bubble and, consequently, release of a drop. In addition to preventing contamination of the liquid by direct contact with the heater 33, the nebulisers 18 prevent excessive heating from causing reactions that might alter substances present in the liquid L.

The number and arrangement of the chambers 30 and the number and arrangement of the nozzles 32 of each chamber 30 may be selected so as to create a uniform cloud of drops, which is desirable for favouring inhalation of the substances present in the liquid L. This is allowed by the freedom of design offered by the semiconductor manufacturing techniques.

In particular, in the microfluidic delivery device 1 the homogeneity of the cloud of drops favours mixing with the air that is drawn in through the inlet holes 13 and released through the mouthpiece 14.

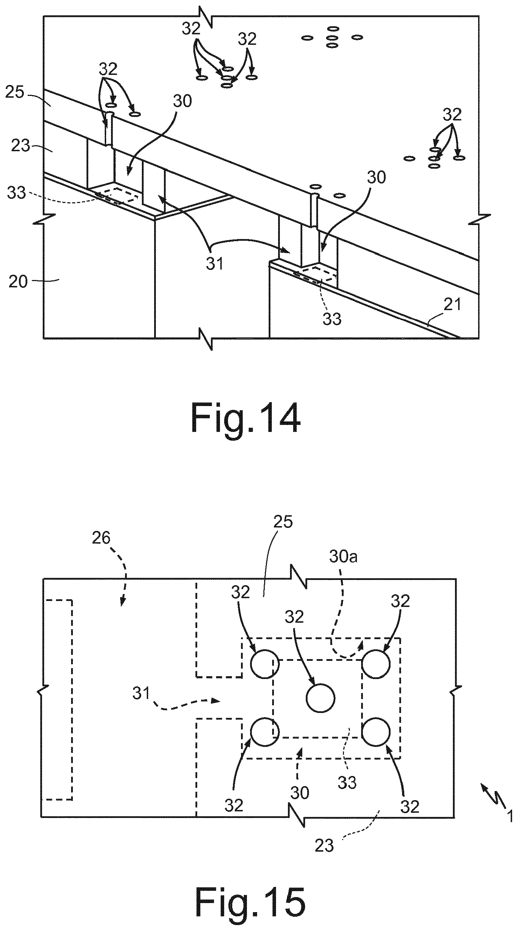

Figures 14 and 15 illustrate an alternative example of arrangement of the nozzles 32. In this case, each chamber 30 is provided with five nozzles 32, one of which is aligned to the centre of the heater 33.

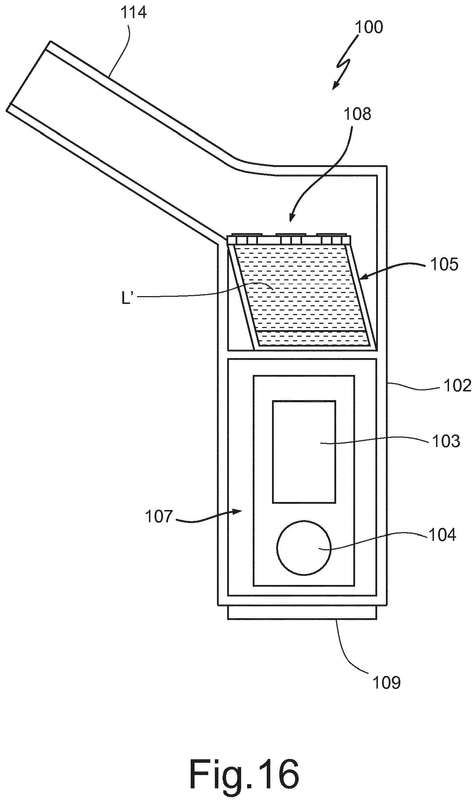

According to a further embodiment (illustrated in Figure 16), a microfluidic delivery device 100, in particular an inhaler for medicinal substances, comprises a casing 102, housed within which are a driving device 103, a battery 104, and a disposable microfluidic cartridge 105. The driving device 103 and the battery 104 are located in a control housing 107, whereas the microfluidic cartridge 105 is located in a cartridge housing 108. The microfluidic cartridge 105 may be made in accordance with of the examples already described previously and contains a liquid L' in which at least one active principle is dissolved in a controlled concentration.

A control pushbutton 109 enables activation of the driving device 103 and causes release of a controlled amount of liquid L' and, consequently, of an equally controlled dosage of active principle. Release is obtained through a mouthpiece 114 integrated in the casing 102. In the example illustrated, no air-inlet holes are provided, and release of the amount of liquid L' is carried out without pre-mixing with a flow of air.

Finally, it is evident that modifications and variations may be made to the microfluidic dispenser device described herein, without thereby departing from the scope of the present invention, as defined in the annexed claims.

## Claims

1. A microfluidic dispenser device for delivering inhalable substances comprising:
a casing (2; 102);
a driving circuit (3; 103), housed in the casing (2; 102);
a microfluidic cartridge (5; 105), housed in the casing (2; 102) and having a tank (17) containing a liquid (L; L') to be delivered;
wherein the microfluidic cartridge (5; 105) is provided with at least one nebuliser (18) controlled by the driving device (3; 103) and comprising:
a substrate (20);
a chamber layer (23) on the substrate (20);
a plurality of chambers (30) formed on the substrate (20) in the chamber layer (23) and fluidically coupled to the tank (17) for receiving the liquid (L; L') to be delivered;
a plurality of heaters (33), which are formed on the substrate (20) in positions corresponding to respective chambers (30), are thermally coupled to the respective chambers (30), and are separated from the respective chambers (30) by an insulating layer (21), the heaters (33) being controlled by the driving device (3); and
at least one nozzle (32) for each chamber (30), the nozzle (32) fluidically connecting the respective chamber (30) to the outside of the nebuliser (18);
wherein the nebuliser (18) comprises supply passages (26) fluidically coupled to the tank (17) and wherein the chambers (30) are fluidically coupled to respective supply passages (26) by means of respective microfluidic channels (31);
wherein the chambers (30) are aligned along edges of the supply passages (26) and are evenly distributed;
**characterized in that** the supply passages (26) are circular and concentric and define annular frame regions (27) that comprise respective portions of the substrate (20), of the insulating layer (21), and of the chamber layer (23) and are also concentric.

2. The device according to Claim 1, comprising a nozzle plate (25) on the chamber layer (23), the nozzles (32) being provided through the nozzle plate (25).

3. The device according to Claim 2, wherein the chambers (30) are delimited by the insulating layer (21) and, on the side opposite to the substrate (20), by the nozzle plate (25).

4. The device according to Claim 2 or Claim 3, wherein the substrate (20) is made of semiconductor material and the chamber layer (23) is made of a polymeric material.

5. The device according to any one of the preceding claims, wherein the chambers (30) are delimited laterally by walls (30a), and the nozzles (32) are provided in positions such that portions of the surfaces of the walls (30a) extend through base areas of at least some of the nozzles (32).

6. The device according to any one of the preceding claims, wherein two outer supply openings (26) define and separate from the rest of the substrate (20) two frame regions (27), and one innermost supply passage (26) is arranged at the centre, and wherein bridges (28) connect the frame regions (27) to one another and the outermost frame region (27) to the substrate (20).

7. The device according to any one of the preceding claims, wherein the nozzles (32) are configured to release drops of liquid having a diameter smaller than 5 µm, in particular 1 µm, in response to activation of the heaters (33) by the driving circuit (3).

8. The device according to any one of the preceding claims, wherein the heaters (33) are made of a material selected from the following group: polycrystalline silicon, Al, Pt, TiN, TiAIN, TaSiN, TiW.

9. The device according to any one of the preceding claims, wherein the microfluidic cartridge (5) has a lid (5a) that closes the tank (17) and wherein the nebuliser (18) is joined to an outer face of the lid (5a) and is fluidically coupled to the tank (17) via through channels (5b) provided in the lid (5a).

10. The device according to any one of the preceding claims, wherein the casing (2; 102) comprises a housing (8; 108) that receives the microfluidic cartridge (5; 105) in a removable way and communicates with the outside through a release mouthpiece (14; 114).

11. The device according to Claim 10, wherein the housing (8) communicates with the outside also through inlet holes (13) and wherein the inlet holes (13) and the mouthpiece (14) are arranged so that suction through the mouthpiece (14) will cause recall of air into the housing (8) through the inlet holes (13), passage of the air drawn in through the housing (8), and subsequent release through the mouthpiece (14).

12. The device according to any one of the preceding claims, wherein the microfluidic cartridge (5) comprises a plurality of nebulizers (18) and a plurality of tanks (17a-17c), separated from each other and configured to contain respective liquids (La-Lc) with respective distinct substances to be delivered, each tank (17a-17c) being fluidly coupled with a respective nebulizer (18) or group of nebulizers (18), distinct from nebulizers (18) or groups of nebulizers (18) of the other tanks (17a-17c).

## Patentansprüche

1. Mikrofluidische Spendervorrichtung zur Abgabe von inhalierbaren Substanzen, umfassend:
eine Umhüllung (2; 102);
eine Antriebsvorrichtung (3; 103), die in der Umhüllung (2; 102) untergebracht ist;
eine mikrofluidische Kartusche (5; 105), die in der Umhüllung (2; 102) untergebracht ist und einen Tank (17) aufweist, der eine abzugebende Flüssigkeit (L; L') enthält; wobei die mikrofluidische Kartusche (5; 105) mit mindestens einem Vernebler (18) versehen ist, der durch die Antriebsvorrichtung (3; 103) gesteuert wird und
umfasst:
ein Substrat (20);
eine Kammerschicht (23) auf dem Substrat (20);
mehrere Kammern (30), die auf dem Substrat (20) in der Kammerschicht (23) ausgebildet sind und zum Aufnehmen der abzugebenden Flüssigkeit (L; L') fluidisch an den Tank (17) gekoppelt sind;
wobei mehrere Heizelemente (33), die auf dem Substrat (20) in Positionen, die jeweiligen Kammern (30) entsprechen, ausgebildet sind, thermisch an die jeweiligen Kammern (30) gekoppelt sind und durch eine Isolierschicht (21) von den jeweiligen Kammern (30) getrennt sind, wobei die Heizelemente (33) durch die Antriebsvorrichtung (3) gesteuert werden; und
mindestens eine Düse (32) für jede Kammer (30), wobei die Düse (32) die jeweilige Kammer (30) fluidisch mit der Außenseite des Verneblers (18) verbindet;
wobei der Vernebler (18) Zufuhrdurchlässe (26) umfasst, die fluidisch an den Tank (17) gekoppelt sind, und wobei die Kammern (30) mittels jeweiligen mikrofluidischen Kanälen (31) fluidisch an jeweilige Zufuhrdurchlässe (26) gekoppelt sind;
wobei die Kammern (30) entlang Rändern der Zufuhrdurchlässe (26) ausgerichtet sind und gleichmäßig verteilt sind;
**dadurch gekennzeichnet, dass** die Zufuhrdurchlässe (26) kreisförmig und konzentrisch sind und ringförmige Rahmenregionen (27) definieren, die jeweilige Abschnitte des Substrats (20), der Isolierschicht (21) und der Kammerschicht (23) umfassen und ebenfalls konzentrisch sind.

2. Vorrichtung nach Anspruch 1, umfassend eine Düsenplatte (25) auf der Kammerschicht (23), wobei die Düsen (32) durch die Düsenplatte (25) bereitgestellt werden.

3. Vorrichtung nach Anspruch 2, wobei die Kammern (30) durch die Isolierschicht (21) und auf der zu dem Substrat (20) entgegengesetzten Seite durch die Düsenplatte (25) abgegrenzt sind.

4. Vorrichtung nach Anspruch 2 oder Anspruch 3, wobei das Substrat (20) aus Halbleitermaterial hergestellt ist und die Kammerschicht (23) aus einem polymeren Material hergestellt ist.

5. Vorrichtung nach einem der vorhergehenden Ansprüche, wobei die Kammern (30) seitlich durch Wände (30a) abgegrenzt sind und die Düsen (32) in Positionen vorgesehen sind, so dass Abschnitte der Oberflächen der Wände (30a) sich durch Basisbereiche von mindestens einigen der Düsen (32) erstrecken.

6. Vorrichtung nach einem der vorhergehenden Ansprüche, wobei zwei äußere Zufuhröffnungen (26) zwei Rahmenregionen (27) definieren und diese von dem Rest des Substrats (20) trennen und ein innerster Zufuhrdurchlass (26) an der Mitte angeordnet ist, und wobei Brücken (28) die Rahmenregionen (27) miteinander und die äußerste Rahmenregion (27) mit dem Substrat (20) verbinden.

7. Vorrichtung nach einem der vorhergehenden Ansprüche, wobei die Düsen (32) dazu konfiguriert sind, Flüssigkeitstropfen mit einem Durchmesser von weniger als 5 µm, insbesondere 1 µm, als Reaktion auf eine Aktivierung der Heizelemente (33) durch die Antriebsvorrichtung (3) freizugeben.

8. Vorrichtung nach einem der vorhergehenden Ansprüche, wobei die Heizelemente (33) aus einem Material hergestellt sind, das ausgewählt ist aus der folgenden Gruppe: polykristallines Silizium, Al, Pt, TiN, TiAlN, TaSiN, TiW.

9. Vorrichtung nach einem der vorhergehenden Ansprüche, wobei die mikrofluidische Kartusche (5) einen Deckel (5a) aufweist, der den Tank (17) schließt, und wobei der Vernebler (18) an eine Außenfläche des Deckels (5a) angefügt ist und mittels Durchgangskanäle (5b), die in dem Deckel (5a) vorgesehen sind, fluidisch an den Tank (17) gekoppelt ist.

10. Vorrichtung nach einem der vorhergehenden Ansprüche, wobei die Umhüllung (2; 102) ein Gehäuse (8; 108) umfasst, da die mikrofluidische Kartusche (5; 105) auf herausnehmbare Weise aufnimmt und durch ein Freisetzungsmundstück (14; 114) mit der Außenseite kommuniziert.

11. Vorrichtung nach Anspruch 10, wobei das Gehäuse (8) außerdem durch Einlassöffnungen (13) mit der Außenseite kommuniziert und wobei die Einlassöffnungen (13) und das Mundstück (14) angeordnet sind, so dass ein Ansaugen durch das Mundstück (14) eine Rückförderung von Luft in das Gehäuse (8) durch die Einlassöffnungen (13), einen Durchlass der eingezogenen Luft durch das Gehäuse (8) und eine anschließende Freisetzung durch das Mundstück (14) bewirken wird.

12. Vorrichtung nach einem der vorhergehenden Ansprüche, wobei die mikrofluidische Kartusche (5) mehrere Vernebler (18) und mehrere Tanks (17a-17c) umfasst, die voneinander getrennt sind und dazu konfiguriert sind, jeweilige Flüssigkeiten (La-Lc) mit jeweiligen verschiedenen abzugebenden Substanzen zu enthalten, wobei jeder Tank (17a-17c) fluidisch mit einem jeweiligen Vernebler (18) oder einer jeweiligen Gruppe von Verneblern (18), die von Verneblern (18) oder Gruppen von Verneblern (18) der anderen Tanks (17a-17c) verschieden sind, gekoppelt ist.

## Revendications

1. Dispositif de distribution microfluidique pour l'administration de substances inhalables, comprenant :
un boîtier (2 ; 102) ;
un dispositif d'attaque (3 ; 103), logé dans le boîtier (2 ; 102) ;
une cartouche microfluidique (5 ; 105), logée dans le boîtier (2 ; 102) et ayant un réservoir (17) contenant un liquide (L ; L') à administrer ;
dans lequel la cartouche microfluidique (5 ; 105) est pourvue d'au moins un nébuliseur (18) commandé par le dispositif d'attaque (3 ; 103) et comprenant :
un substrat (20) ;
une couche à chambres (23) sur le substrat (20) ;
une pluralité de chambres (30) formées sur le substrat (20) dans la couche à chambres (23) et en communication fluidique avec le réservoir (17) en vue de recevoir le liquide (L ; L') à administrer ;
une pluralité d'éléments chauffants (33), qui sont formés sur le substrat (20) à des positions correspondant à des chambres (30) respectives, sont couplés thermiquement aux chambres (30) respectives, et sont séparés des chambres (30) respectives par une couche isolante (21), les éléments chauffants (33) étant commandés par le dispositif d'attaque (3) ; et
au moins une buse (32) pour chaque chambre (30), la buse (32) mettant la chambre (30) respective en communication fluidique avec l'extérieur du nébuliseur (18) ;
dans lequel le nébuliseur (18) comprend des passages d'alimentation (26) en communication fluidique avec le réservoir (17) et dans lequel les chambres (30) sont en communication fluidique avec des passages d'alimentation (26) respectifs au moyen de canaux microfluidiques (31) respectifs ;
dans lequel les chambres (30) sont alignées le long de bords des passages d'alimentation (26) et sont réparties régulièrement ;
**caractérisé en ce que** les passages d'alimentation (26) sont circulaires et concentriques et définissent des régions de cadre (27) annulaires qui comprennent des parties respectives du substrat (20), de la couche isolante (21) et de la couche à chambres (23), et sont également concentriques.

2. Dispositif selon la revendication 1, comprenant une plaque à buses (25) sur la couche à chambres (23), les buses (32) étant ménagées à travers la plaque à buses (25).

3. Dispositif selon la revendication 2, dans lequel les chambres (30) sont délimitées par la couche isolante (21) et, du côté opposé au substrat (20), par la plaque à buses (25).

4. Dispositif selon la revendication 2 ou la revendication 3, dans lequel le substrat (20) est fait d'un matériau semi-conducteur et la couche à chambres (23) est faite d'un matériau polymère.

5. Dispositif selon l'une quelconque des revendications précédentes, dans lequel les chambres (30) sont délimitées latéralement par des parois (30a), et les buses (32) sont ménagées à des positions telles que des parties des surfaces des parois (30a) s'étendent à travers des zones de base d'au moins certaines des buses (32).

6. Dispositif selon l'une quelconque des revendications précédentes, dans lequel deux ouvertures d'alimentation (26) extérieures définissent et séparent du reste du substrat (20) deux régions de cadre (27), et un passage d'alimentation (26) le plus à l'intérieur est agencé au centre, et dans lequel des ponts (28) relient les régions de cadre (27) l'une à l'autre et la région de cadre (27) la plus à l'extérieur au substrat (20).

7. Dispositif selon l'une quelconque des revendications précédentes, dans lequel les buses (32) sont configurées pour délivrer des gouttes de liquide ayant un diamètre inférieur à 5 µm, en particulier de 1 µm, en réponse à une activation des éléments chauffants (33) par le dispositif d'attaque (3).

8. Dispositif selon l'une quelconque des revendications précédentes, dans lequel les éléments chauffants (33) sont faits d'un matériau sélectionné dans le groupe suivant : silicium polycristallin, Al, Pt, TiN, TiAlN, TaSiN, TiW.

9. Dispositif selon l'une quelconque des revendications précédentes, dans lequel la cartouche microfluidique (5) a un couvercle (5a) qui ferme le réservoir (17) et dans lequel le nébuliseur (18) est assemblé à une face extérieure du couvercle (5a) et est en communication fluidique avec le réservoir (17) par l'intermédiaire de canaux traversants (5b) ménagés dans le couvercle (5a).

10. Dispositif selon l'une quelconque des revendications précédentes, dans lequel le boîtier (2 ; 102) comprend un logement (8 ; 108) qui reçoit la cartouche microfluidique (5 ; 105) d'une manière amovible et communique avec l'extérieur par un embout buccal de sortie (14 ; 114).

11. Dispositif selon la revendication 10, dans lequel le logement (8) communique avec l'extérieur également par des trous d'admission (13) et dans lequel les trous d'admission (13) et l'embout buccal (14) sont agencés de manière qu'une aspiration par l'embout buccal (14) provoque un appel d'air dans le logement (8) par les trous d'admission (13), un passage de l'air aspiré à travers le logement (8), et une sortie subséquente par l'embout buccal (14).

12. Dispositif selon l'une quelconque des revendications précédentes, dans lequel la cartouche microfluidique (5) comprend une pluralité de nébuliseurs (18) et une pluralité de réservoirs (17a-17c), séparés l'un de l'autre et configurés pour contenir des liquides (La-Lc) respectifs comprenant des substances distinctes respectives à administrer, chaque réservoir (17a-17c) étant en communication fluidique avec un nébuliseur (18) ou groupe de nébuliseurs (18) respectif, distinct de nébuliseurs (18) ou groupes de nébuliseurs (18) des autres réservoirs (17a-17c).
